# EUROPEAN PATENT SPECIFICATION

(11) **EP 3 512 472 B1**
(45) Date of publication and mention of the grant of the patent: **28.02.2024**
(21) Application number: 17768781.1
(22) Date of filing: 15.09.2017
(51) Int. Cl.: A61F 5/443, A61F 5/451

(54) **FAECAL COLLECTING SYSTEM**
SAMMELSYSTEM FÜR EXKREMENTE
SYSTEME DE COLLECTION POUR EXCREMENTS

(30) Priority: 16.09.2016 DK PA201600545
(43) Date of publication of application: 24.07.2019
(73) Proprietor: Furine ApS, 3400 Hillerød (DK)
(72) Inventor: NIELSEN, Brian, 3330 Gørløse (DK)
(74) Representative: Guardian IP Consulting I/S
(86) International application number: PCT/EP2017/073345
(87) International publication number: WO 2018/050856

(56) References cited:
- EP-A1- 2 803 342
- DE-C- 79 818
- US-A- 5 421 827
- US-A1- 2006 095 011
- US-A1- 2009 227 970

## Description

The present invention relates to a faecal collecting system arranged to collect faecal waste from a user. The system comprises: an attachment member arranged to be attached to the user, said attachment member comprising an opening which is arranged to be in fluid communication with the anus of the user and an attachment flange which is arranged to encircle said opening and which is arranged to be attached to the peri-anal skin surrounding the anus of the user such that a fluid tight seal is established between the attachment flange and the peri-anal skin of the user entirely around the anus of the user, a collection bag for collecting the faecal output, and a hollow conduit having a first end and a second end, said first end connected to said attachment member and being in fluid communication with the opening of said attachment member and said second end being in fluid communication with said collection bag.

### Description of related art

Many faecal collecting systems are disclosed in the prior art. Some are arranged as collecting bags attached directly to the peri-anal skin and some are arranged as catheter based systems, where a tube is inserted directly into the anal canal itself. Examples of catheter based systems are disclosed in US2003/0073963 and US20060129135. Typically, catheter based systems, are fastened inside the anus via an inflatable balloon. These systems may drop out of the rectum if there is not enough pressure in the balloon or if they are inserted incorrectly. Correct placement is difficult to judge since the holding mechanism is arranged inside the rectum of the user. Furthermore, they may harm the rectum if the balloon explodes or is inflated too much. They are also uncomfortable to wear over time. Catheter based systems are sometimes called anal irrigation devices as they are usually provided with fluid introducing elements which can introduce fluid into the anus to soften hard stool inside the anus, making it possible for the stool to leave the anus via the catheter tube. A variation on the catheter based system is disclosed in DE79818. In this document, it is desired to introduce an external attachment device to the rectal area of the user via an external strap system. However this is very difficult to make fluid tight and is uncomfortable for the user.

Examples of collecting bag based systems are disclosed in US20060095011, US3522808, US3577989, CN202096328U, CN202776688U, EP2803342, US2625160A, CN201453459U, WO2009021520A1, US2012245544A1, US4784656A and US5312384A. The prior art systems typically disclose faecal collecting systems where the collecting bag is located just outside the anal opening. This creates discomfort for the user. Furthermore there is a risk that when the user moves, the user can compress the collecting bag or cause the collecting bag to become detached from the body of the user. This can cause undesired leakage.

Furthermore, collecting bags are typically fastened to the peri-anal skin via skin adhesives. These devices may be inconvenient to use and/or not be sufficiently leakage proof. Likewise, frequent changing of the device leads to discomfort to the user since the adhesive needs to be removed and re-applied frequently. This furthermore results in an inefficient cost structure due to frequent need for changing the entire system.

Another bag-based system is disclosed in US5421827. This system is unique in that it has an applicator inside the bag to help apply the system to the user. It has however been shown to be difficult to use the applicator in practice and the seal created by the bag to the rectal area of the user can develop leaks.

There therefore remains a need for a system with an external fixation device, which is easy to apply, but which is also more leakage proof and more comfortable for the user. Furthermore, there remains a need for a system where the individual parts can be changed at different times/intervals to reduce the treatment cost and reduce discomfort for the user. For example, on external faecal collecting systems which comprise an attachment element (typically with a skin adhesive) and a collecting bag, it could be a benefit to change the collecting bag at a different time interval than changing the adhesive. For example, the attachment element attached to the user via an adhesive could be changed every 7th day while the collecting bag could be changed a couple of times a day. This would reduce the overall treatment cost and reduce discomfort for the user. Furthermore, if one part had a malfunction, only that one part would need to be changed.

The present invention is in particular useful on users that have relatively liquid faecal output. One example of such users are patients which are being given substantial medical treatment, for example patients in intensive care units. Document DE 79 818 C discloses a faecal collecting system considered as representing the closest prior art.

### Summary of the invention

The above mentioned problems are solved at least in part with a faecal collection system as mentioned in the introductory paragraph and as characterized in claim 1.

In this way, a system is provided which is simple to apply, easy to exchange, comfortable to wear and with a low risk of leakage. By choosing the average diameter of the conduit to be less than 8cm, the conduit does not fill with so much faeces that it becomes heavy and uncomfortable to the user. Furthermore, if leakage does occur, there is not so much faeces in the conduit which can leak out. Likewise, by making the conduit more than 30cm long, the collecting bag is placed at a distance from the user, thereby again reducing the discomfort for the user and reducing the risk of leakage.

In other embodiments, the conduit could have an average diameter which is less than 5cm, less than 4cm or less than 2,5 cm. In certain embodiments, the length of the conduit could be greater than 40cm, greater than 50cm or greater than 60cm.

In one embodiment of the system, the conduit could be provided with anti-kinking and/or anti-collapsing means. By anti-kinking means is meant means which prevent the conduit from kinking and thereby blocking the flow through the conduit. Anti-collapsing means are understood as means which prevent the opposing side walls of the conduit from coming into contact with each other which could block the flow of stool through the conduit. It should be understood that such means can be formed in different forms and with different preventive qualities. In one example, a soft flexible conduit could be provided with a slight spiral ridge extending along the length of the conduit to strengthen the side walls. Of course, it would still be possible to kink this conduit and/or force the walls to collapse, but the spiral will still to some extent reduce the risk of kinking and/or collapsing.

In one embodiment, the attachment flange could have an outer width or diameter which is larger than the width or diameter of the conduit near the first end of the conduit. In this way, the outer edges of the attachment flange would extend outwardly past the outer sides of the conduit. In this way, it will be possible to apply force to the attachment flange from the outside of the conduit. This will help in pressing the attachment flange into contact with the skin of the user.

In order to allow the collection bag to be exchanged without removing the attachment member from the user, the system could be provided with a conduit connection member which allows the conduit to be detachably connected to the collection bag. In one embodiment, the conduit connection member could be arranged on the conduit and be detachably connectable to a connection member which is in fluid communication with the collection bag. Typically such a conduit connection member would be placed at the second end of the conduit, however, the conduit connection member could also be placed in the middle of the conduit such that a portion of the conduit was attached to the collection bag and a portion was attached to the attachment member. Likewise, one could imagine an embodiment where the conduit connection member was attached close to the attachment member, so the majority of the conduit was exchanged when the collection bag was exchanged. Other embodiments could be imagined where a conduit connection member is placed at both ends of the conduit so that the conduit could also be exchanged independently of the attachment member and the collection bag. In one embodiment, a hose fitting is placed on the collection bag and the end of the conduit is pressed into the fitting.

In order to improve the sealing abilities of the attachment member, the attachment flange could be provided with a skin friendly adhesive on a skin contacting surface of said attachment flange.

In cases where an adhesive is used, the adhesive could be provided separately and applied just prior to use, or it could be provided already applied on the flange portion of the attachment member prior to packaging. Some skin friendly adhesives which could be used are adhesives such as Acrylic, Hydrocolloid and Silicone adhesives.

Some adhesive systems, such as Hydrocolloid adhesives, are arranged as discrete molecules on multiple networks. These systems have a tendency to creep and do not remember their original cast shape. Other adhesives systems are cured into, in principle, one large molecular network. These systems have some degree of shape-memory and want to return to their original cast shape. Depending on the application, one or the other system might be preferred.

In one embodiment, the adhesive could be an uncured curable liquid adhesive. In one beneficial embodiment which uses adhesive, the attachment flange could be provided with a groove on the skin contacting surface of said attachment flange, said groove encircling the opening, and said groove being filled with an uncured curable liquid adhesive. By providing a groove having a certain depth, a greater amount of liquid adhesive could be applied. This will increase the amount of adhesive between the attachment flange and the skin and thereby allow the adhesive to flex and adapt to the movements of the user better. More adhesive will also seal against more surface area of the user and thereby establish a better seal.

In one embodiment, the adhesive is provided separately from the attachment member and applied to the attachment member prior to use. In one embodiment, the adhesive is an uncured curable liquid adhesive which is packaged separately from the attachment flange. In one embodiment of the system, the system is arranged as a kit of parts comprising a conduit, a collecting bag and an attachment member and an adhesive, where the adhesive is separately packaged from the attachment member.

In one embodiment, the adhesive could be formed as a cured adhesive.

In one embodiment, the adhesive is applied to the attachment member prior to packaging. When applying the adhesive to the attachment member prior to packaging, it can be desired to use an adhesive with low creep, for example a cured adhesive, as it might otherwise partly flow away from its desired position between packaging and use.

In one embodiment the attachment member comprises an adhesive which exercises shape memory. In one embodiment, the attachment member comprises an adhesive which is cast into a specific stable shape prior to packaging.

Since the perianal anatomy varies quite a bit between different human beings, a mass production of attachment members fitting each different anatomy is not economically feasible. Hence, the flange portion and/or the adhesive should be sufficiently flexible and elastic to be able to adapt to the varying anatomies.

In one embodiment, the attachment flange comprises a first portion closest to the opening of the attachment member and a second portion further away from the opening than the first portion, said first portion being stiffer than said second portion. In one embodiment, an adhesive covers the skin facing surface of both the first and second portions. In one embodiment, the first portion has a first thickness and the second portion has a second thickness, said second thickness being less than said first thickness. In one embodiment, the first portion is formed of a silicon moulded material or other plastic moulded material and the second portion is formed of a nonwoven sheet or other pliable foil material having a stiffness which is less than the first portion.

In one embodiment, the dimension of the first portion in a direction parallel to the plane of the first portion from the opening to the outward edge of the first portion is less than the dimension in a direction parallel to the plane of the second portion from the outward edge of the first portion to the outward edge of the second portion. In one embodiment, said dimensions of the second portion at the ventral and dorsal ends of the attachment member are greater than said dimension at the sides of the attachment member.

In one embodiment, the opening of the attachment member could be formed with an oval shape. This will allow the attachment member to fit better between the buttocks of the user while still maintaining a suitably sized opening. Likewise, the attachment flange could be formed with an oval shape. In one embodiment the attachment flange and/or the opening has a length (in the dorsal/ventral direction) which is greater than the width.

In one embodiment, the attachment flange could be formed as a 3D formed structure, in contrast to a traditional planar flange.

In one embodiment, the attachment flange is provided in a saddle like shape.

In one embodiment, the dorsal and/or ventral portion(s) of the attachment flange is/are higher than the central portion of the attachment flange. In one embodiment, the height difference is at least 3mm, at least 5mm or at least 7mm.

In one embodiment, the attachment flange is provided with an adhesive on the skin facing surface, said adhesive being formed into a 3D formed structure. In one embodiment, the thickness of the adhesive layer is greater than 1 mm, greater than 2mm or greater than 3mm.

While the buttocks effectively support the adhesive seal on the side, it has surprisingly been found that it is much more difficult to create a fluid tight seal between the attachment member and the skin at the Perineum and Crena Ani. Therefore in one embodiment, the dorsal and/or ventral portion of the attachment flange are given a significant surface area in order to ensure a larger adhesion surface at the Perineum and Crena Ani.

In one embodiment, the dorsal and/or ventral portion(s) of the attachment flange extend dorsally at least 12 mm, at least 15mm, at least 18mm or at least 20mm from the dorsal/ventral portion of the opening respectively. In one embodiment, the attachment flange extends at least 12mm, at least 15mm, at least 18mm or at least 20mm from the opening around the entire circumference of the opening.

In one embodiment, the system could further comprise an applicator, said applicator comprising a handle portion, an opening arranged at one end of the handle portion, said opening being arranged to allow the conduit to pass through the opening and a pressure applying surface arranged at the opening, said pressure applying surface being arranged to be suitable for pressing the attachment flange of the attachment member against the perianal skin of the Perineum and the Crena Ani of the user.

Such an applicator could be provided as one component of a kit of parts comprising an applicator, a conduit, a collecting bag and an attachment member. Such an applicator could also be provided as a kit of parts comprising an applicator and an attachment member or a kit of parts comprising an applicator, an attachment member and a conduit. These kits of parts could also comprise a fastening structure as described later on and/or an adhesive. In one embodiment, the pressure applying surface could be arranged to be suitable for pressing the attachment flange of the attachment member against the perianal skin surrounding the anus of the user.

In one embodiment, the handle portion could be provided with a hollow portion inside the handle portion and said opening could be arranged at one end of the hollow portion such that the conduit can pass through the opening and into the hollow portion inside the handle portion

In one embodiment, the pressure applying surface of the applicator could be arranged to completely encircle the conduit during application of the attachment flange to the user. In this way, the pressure applying surface could press uniformly against the perianal skin, all the way around the anus of the user. In one embodiment, the pressure applying surface of the applicator could be arranged primarily at the dorsal and ventral portion of the applicator to ensure that at least the dorsal and ventral portions of the attachment flange are pressed into firm contact with the perianal skin at the Perineum and Crena Ani of the user.

When applying the attachment member to the perianal skin, and especially in the case where an adhesive is used, it may be important that the skin facing surface of the attachment flange is, after application, in full contact with the perianal skin and that there are no internal residual stresses in the attachment flange due to the application to the skin. Therefore, it may be important that the applicator support the position of the adhesive on all of the perianal skin surrounding the anus. Since the perianal skin typically has a shape opposite to a saddle, where the anal orifice is located at the lowest point, the pressure applying surface of the applicator could advantageously have a shape similar to a saddle.

In one embodiment, the pressure applying surface could be formed as a 3D surface. In one embodiment, dorsal and/or ventral portion(s) of the pressure applying surface could be arranged higher than a middle portion of the pressure applying surface.

In one embodiment, dorsal and/or ventral portion(s) of the pressure applying surface of the applicator could have a top point elevated at least 5 mm, at least 7mm, at least 9mm or at least 11 mm in the cranial direction from the portion of the applicator adapted to receive the opening of said attachment member.

The perianal anatomy of the users are of a relative narrow shape both ventrally (in Perineum) and dorsally (in Crena Ani). Still the attachment flange and/or the adhesive should engage completely with the peri-anal skin both to Crena Ani and to Perineum. The Care giver compensates in part by manual separation of the buttocks, but it may furthermore also be supported by the top shape of the Applicator being formed relative thin ventrally and/or dorsally.

In one embodiment, the ventral and/or dorsal shapes of the applicator could be narrow, so that the applicator can press the attachment flange and/or the adhesive into full contact with the perineum and/or Crena Ani. In one embodiment, the ventral and dorsal portions of the pressure applying surface could be greater than 2mm wide, greater than 3mm wide or greater than 4mm wide. In one embodiment, the ventral and dorsal portions of the pressure applying surface could be less than 13mm wide, less than 11 mm wide, less than 8mm wide or less than 6mm wide. Any combinations of these values could be used.

While the buttocks support the adhesive seal on the side, it has surprisingly been found that it is much more difficult to create a fluid tight seal between the attachment member and the skin at the Perineum and Crena Ani. Therefore in one embodiment, the dorsal and/or ventral portion of the pressure applying surface of the applicator are given a significant length in order to ensure a larger surface of interaction between the pressure applying surface of the applicator and the perianal skin of the user at the Perineum and Crena Ani via the attachment flange of the attachment member.

In one embodiment, the dimension of the dorsal and/or ventral portion of the pressure applying surface of the applicator which extends from the opening of the applicator adapted to receive the attachment member to the most dorsal/ventral extent of the pressure applying surface is at least 13mm, at least 15mm, at least 17mm or at least 20 mm.

In one embodiment, the angle between the path along the dorsal portion of the pressure applying surface along the central plane of the applicator and the path along the ventral portion of the pressure applying surface along the central plane of the applicator is less than 170 degrees, less than 155 degrees, or less than 140 degrees. In this way, the pressure applying surface will better fit the shape of the perianal skin and especially the form of the Perineum and the Crena Ani.

In one embodiment, the pressure applying surface could be arranged to be openable so that the pressure applying surface no longer completely encircles the conduit when the pressure applying surface is opened. In this way, the applicator can be removed from the conduit without having to pull the applicator all the way to the end of the conduit. In one embodiment, the applicator could be formed of two portions which are clicked together along a parting line which runs from the first opening to the side of the handle portion opposite the first opening and which can be unclicked once the attachment member is properly attached to the user.

In one embodiment, the applicator could be arranged such that the conduit can be arranged and/or packaged inside said hollow portion. In this way, the applicator could be formed as part of the packaging for the attachment member and/or the conduit and/or the collecting bag.

In one embodiment, the connection member could be releaseably attached to the applicator.

In one embodiment, the system could comprise a fastening structure, said fastening structure comprising a waistband and a supporting strap arrangement arranged to press the attachment flange of the attachment member against the peri-anal skin of the user. In this way, the attachment member could be maintained in position via a mechanical fastening, thereby reducing the need for adhesive.

In one embodiment, the fastening structure could comprise at least one groin strap and the attachment member could comprise at least one receiving element for receiving said groin strap.

In one embodiment, the system could comprise both a fastening structure and an adhesive. In this case, the adhesive could provide good sealing properties, but provide a less strong adhesive effect since the fastening structure would provide the fastening effect. Using a very flexible but less strong adhesive would increase the sealing properties, while also making it easy to exchange the system without discomfort to the user. However, in this case, a fastening structure would be necessary to provide an effective attachment to the user, since the adhesive might not be secure enough by itself. This combination could form the basis for a divisional application directed to a system comprising an attachment member and a collection bag as previously described and further comprising anadhesive applied on the attachment flange and a fastening structure as described above. The combination of adhesive and fastening structure provides for a unique system which is easy to apply and comfortable to wear and exchange.

In one embodiment, the applicator could comprise two parts, a first part which is pressed against the attachment flange and a handle portion to properly manipulate the first part. The handle portion could then be disconnected from the first part, leaving the first part in contact with the attachment flange. In one embodiment, the first part could be provided with a receiving element which can receive the straps of a fastening structure.

In one embodiment the fastening structure could comprise a supporting element which applies pressure to the attachment flange at the ventral and/or dorsal portions of the attachment flange. In this way, the attachment flange will be pressed into contact with the Perineum and Crena Ani portions of the user.

It should also be noted that the current specification discloses at least three separate inventions. The first invention is described in the current claim set and relates to a system having a conduit which is at least 30cm long and has an average diameter of less than 8cm. A second invention relates toan external applicator to apply the attachment member to the peri-anal skin of a user. This second invention is specified in more detail below. This second invention could form the basis of a divisional application in the future. The third invention is described above and comprises an attachment member with adhesive and a fastening structure. This third invention could also form the basis of a divisional application in the future.

Second invention: Faecal collecting system arranged to collect faecal waste from a user, said system comprising: an attachment member arranged to be attached to the user and a collection bag for collecting the faecal output, said attachment member comprising an opening which is arranged to be in fluid communication with the anus of the user and an attachment flange which is arranged to encircle said opening and which is arranged to be attached to the peri-anal skin surrounding the anus of the user such that a fluid tight seal is established between the attachment flange and the peri-anal skin of the user entirely around the anus of the user, said collecting bag being in fluid communication with the opening in the attachment member, said system further comprising an applicator, said applicator comprising a handle portion, an opening arranged at one end of the handle portion, said opening being arranged to allow a conduit connecting the attachment member to the collection bag to pass through the opening and a pressure applying surface arranged at the opening, said pressure applying surface being arranged to be suitable for pressing the attachment flange of the attachment member against the peri-anal skin at the Perineum and at the Crena Ani of the user.

It should be noted that unless specifically mentioned, the form of the conduit in this second invention is not further defined. In one embodiment the conduit could be a short, ie less than 30cm conduit. In another embodiment the conduit could be an integral part of the attachment member. In another embodiment the conduit could be an integral portion of the collection bag. In another embodiment, the attachment member could be an integral portion of the collection bag.

In one embodiment, the applicator could comprise;
1) a ventral top point (A) on a ventral portion of the pressure applying surface;
2) a dorsal top point (B) on a dorsal portion of the pressure applying surface;
3) a middle top point on a middle portion of the pressure applying surface in the ventral-dorsal direction (C), said middle top point being lower than the dorsal top point and/or the ventral top point; and where
the applicator is characterized in that the angle formed between the two lines CA and CB is between 115 and 170 degrees, between 120 and 160 degrees, between 125 and 150 degrees or any combination between these intervals.

In one embodiment, the applicator is arranged such that the pressure applying surface surrounds the entire opening and the pressure applying surface is arranged to be suitable for pressing the attachment flange of the attachment member against the peri-anal skin surrounding the anus of the user.

Additional embodiments could be formed by combining the features of the different embodiments of the first invention as described above and in the claims with the above presented definition of the second invention and/or the third invention. It is clear that the person skilled in the art when presented with the different features of the different embodiments will be able to combine the different features into different embodiments. All the different combinations will therefore not be listed here. In particular, it should be noted that the features related to specific shapes of the attachment member/flange and/or the specific shapes of the pressure applying surface of the applicator can be combined as desired into new claims.

It should be emphasized that the term "comprises/comprising/comprised of" when used in this specification is taken to specify the presence of stated features, integers, steps or components but does not preclude the presence or addition of one or more other features, integers, steps, components or groups thereof. For example, in the claims it is specified that the system comprises a flexible conduit. However, within the scope of the claims, it should be clear that the system could comprise additional flexible conduits.

### Brief description of the drawings

In the following, the invention will be described in greater detail with reference to embodiments shown by the enclosed figures. It should be emphasized that the embodiments shown are used for example purposes only and should not be used to limit the scope of the invention.
Figure 1 schematically shows a general overview of the different components of a faecal collecting system according to the current invention.
Figure 2 discloses a schematic cross section view of an embodiment of a system according to the present invention.
Figures 3 and 4 disclose a side view and a top view respectively of one component of the system of figure 2.
Figure 5 shows a 3-dimensional view of a fastening structure for securing the system of figure 2 to a user.
Figure 6 discloses a 3-dimensional view of another embodiment of a faecal collecting system according to the present invention.
Figure 7 discloses a cross sectional view of an embodiment of an attachment member.
Figure 8 discloses a fastening structure connected to the attachment member of figure 7.
Figures 9a to 9b disclose schematic cross sectional and top views respectively of an embodiment of an attachment member according to the present invention.
Figures 10a-10b disclose schematic cross sectional and top views respectively of an attachment member with adhesive.
Figure 11 discloses a schematic cross sectional view of an attachment member and an applicator.
Figure 12 discloses a schematic cross sectional view of another attachment member and another applicator.
Figure 13a and 13b disclose schematic cross sectional views of another attachment member and applicator and two steps of the procedure for attaching the attachment member to the user.
Figure 14 discloses a schematic view of a kit of parts comprising an attachment member according to the invention, an applicator and an adhesive member.
Figure 15 discloses a schematic cross sectional view of the attachment member of figure 14.
Figures 16a-16d schematically show four different steps in the process of attaching the attachment member of figure 14 to a user.
Figure 17a discloses a front view of one embodiment of an attachment member.
Figure 17b discloses a side view of the attachment member of figure 17a.
Figure 17c discloses a top view of the attachment member of figure 17a.
Figure 18a shows a top front perspective view of an applicator suitable for applying the attachment member of figures 17a-17c.
Figure 18b shows a bottom front perspective view of the applicator of figure 18a.
Figure 18c shows a front view of the applicator of figure 18a.
Figure 18d shows a side view of the applicator of figure 18a.
Figure 18e shows a top view of the applicator of figure 18a.
Figure 18f shows a bottom view of the applicator of figure 18a.
Figure 19 shows a perspective view of an embodiment of an applicator and an attachment member.
Figure 20 shows an exploded perspective view of the applicator and attachment member of figure 19.
Figure 21 shows a cross sectional view of the applicator and attachment member shown in figure 19.
Figure 22 shows an exploded cross sectional view of figure 21.
Figure 23 shows an exploded side view of the applicator and attachment member of figure 19.
Figure 24 shows a partial cross sectional view through the longitudinal plane of the applicator and attachment member with an adhesive applied.
Figure 25 shows a cross section view through the transverse plane of the attachment member with adhesive applied.

### Detailed description of the embodiments

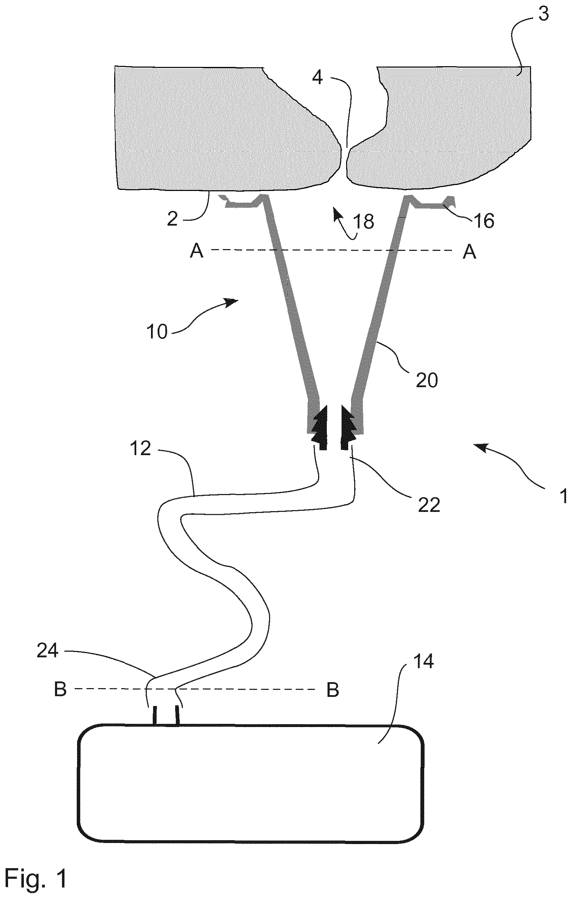

Figure 1 discloses a very schematic illustration showing the main components of the faecal collecting system according to the current invention when attached to a user. The system 1 is arranged to be attached to the peri-anal skin 2 of a user 3 around the anal orifice 4 and collect faecal waste exiting the anus of the user. The system 1 in general comprises an attachment member 10, a conduit 12 and a collection bag 14. The attachment member is arranged to establish a fluid tight connection between the system and the user. The conduit is arranged to establish fluid communication between the attachment member and the collection bag. The collection bag is arranged to collect the faecal output.

The attachment member 10 comprises an attachment flange 16 which is attached to the peri-anal skin of the user. The attachment member 10 of the current embodiment also comprises an opening 18 and an elongated portion 20. The conduit 12 has two ends, a first end 22 in fluid communication with the attachment member and a second end 24 in fluid communication with the collection bag.

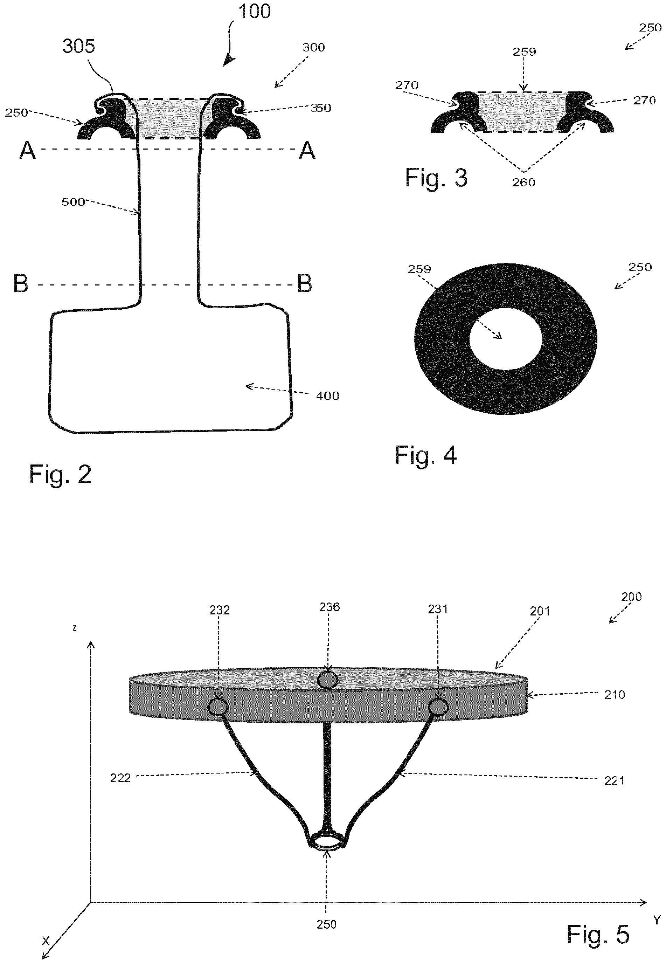

In the current schematic embodiment, there are three separate elements, an attachment member, a conduit and a collection bag. However, with the scope of the invention, these elements could be integrated together. For example, an embodiment could be made which comprised a single element. This is shown in relation to the embodiment shown in figures 2-5. In another embodiment, the conduit and attachment member could be integrated into a single element, or the conduit and the collection bag could be integrated into a single element. Likewise, it should be mentioned that the conduit and the elongated portion of the attachment member fulfil similar functions. For the sake of the current specification and for the understanding of the claims, the attachment member should be understood as the part of the system which establishes a fluid tight communication with the anus of the user, the conduit should be understood as that part of the system which establish fluid communication between the attachment member and the collection bag, and the collection bag is the part of the system which collects the faecal waste from the user.

In the embodiment shown in figure 1, it can therefore be understood that the portion of the system which is above the line AA is considered the attachment member, the portion which lies under the line BB is considered the collection bag and the portion which lies between the lines AA and BB is considered the conduit. Using this understanding, according to the claims the conduit should be at least 30 cm long and less than 8 cm in diameter. When compared to prior art solutions, this is longer and/or thinner than any systems which are attached externally to the perianal skin.

In the current embodiment shown in figure 1, the attachment flange 16 is attached to the peri-anal skin via an adhesive 26 applied between the attachment flange and the peri-anal skin. The adhesive is discussed in more detail later on in this specification. In another embodiment, as will be discussed later on, instead of an adhesive, a mechanical system could be used with straps to press the attachment flange against the peri-anal skin. Adhesive and mechanical systems can be combined if desired.

Figures 2-5 disclose different views of different elements/parts of a second embodiment of a faecal collecting system according to the current invention. The system comprises a one piece integrated flexible plastic element 100 made from a sheet/film material and comprising a collection bag 400, a conduit 500 and an attachment flange 310. The attachment flange 305 is pressed into contact with the peri-anal skin of the user member by an annular ring 250 which presses upwardly against the surface of the attachment flange which faces away from the peri-anal skin of the user. An opening 259 is formed in the annular ring 250. In use, the opening 259 of the annular ring is aligned with the anal orifice of the user. The conduit 500 is in this case arranged as a tubular section which is also aligned with the opening in the annular ring and the anal orifice of the user.

In this embodiment, the conduit 500 is provided as a flexible elastic sheet tubular sheet element. The end of the tubular sheet element is formed with a thickened portion 350. Prior to use, the thickened portion 350 of the conduit is inserted through the opening in the annular ring and then expanded outwardly such that it can be applied over the upper edge of the annular ring. The thickened portion is then, in this embodiment, arranged in a groove 270 in the outer surface of the annular ring. In this way, an attachment flange 310 is formed which can be pressed into contact with the peri-anal skin of the user.

Figure 3 shows a cross sectional view of the annular ring 250. The annular ring 250 has a ring shaped form with an opening 259 adapted to encircle the orifice of the anus of the user. The annular ring 250 has a receiving portion 260 on its left and right side for receiving a portion of a fastening structure 201, see figure 2. The annular ring 250 also comprises a fastening portion 270 as described above.

Figure 5 discloses a 3-dimensional view of one example of a fastening structure 201 suitable for attaching the annular ring to the user. The fastening structure 201 comprises a waist band 210, a left groin string 221 and a right groin string 222. On this example the groin strings 221, 222 are elastic circular strings. The groin strings 221, 222 are attached to the waist band 210 both ventrally 531, 532 and dorsally 236. The annular ring 250 is shown attached to the groin strings 221,222. Prior to attachment to a user, the conduit 500 should be inserted through the opening 259 of the ring and then attached to the ring.

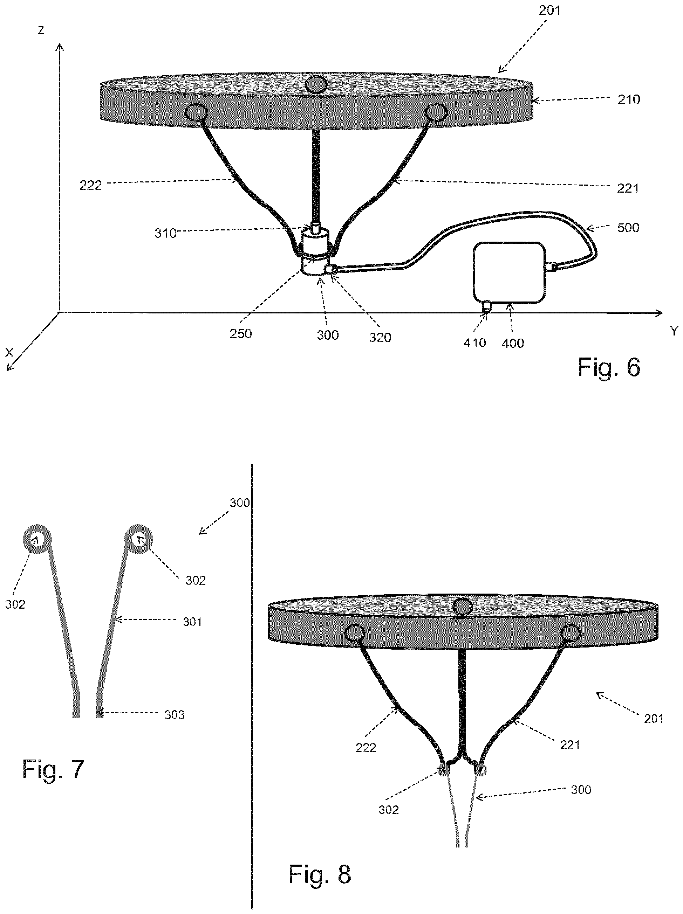

Fig. 6 discloses a 3-dimensional view of an example of a faecal management system comprising a fastening structure 201 similar to the one shown in figure 5. However, in this case, instead of being connected to a flexible sheet conduit as in figure 2, the system in this example shows an anal irrigation device. The attachment member 300 in this embodiment comprises one inlet 310 and one outlet 320, the outlet being connected to a collecting bag 400 via a tube 500. The attachment member 300 is supported in place via the annular ring 250, as in the previous embodiment. The bag 400 comprises an opening 410 for occasionally emptying the bag 400.

Figures 7 and 8 disclose a slightly different version of the attachment member. In this embodiment, the attachment member 300 is formed of a plastic flexible material having an elongated portion 301 and reinforced upper edge 302. The upper edge is stiff enough to be supported directly by the groin straps without the need for the annular ring of the previous embodiment. Likewise, the reinforced upper edge 302 also functions as the attachment flange which is pressed into contact with the peri-anal skin of the user.

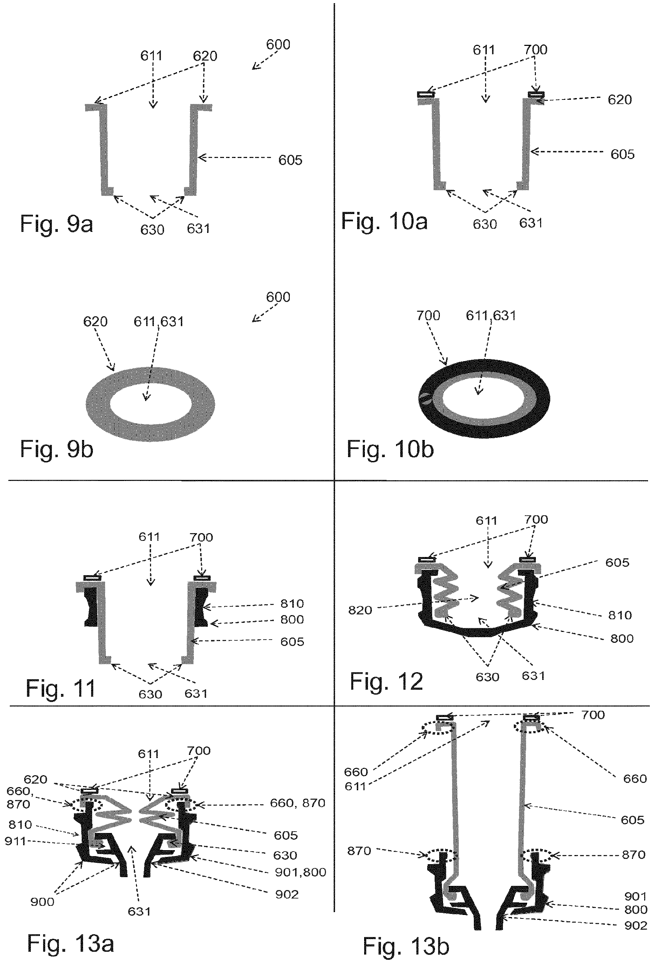

Figures 9a and 9b disclose views of an example of a hollow attachment member 600 according to the present invention. Fig. 9a discloses a cross sectional view of the hollow attachment member 600 comprising the first opening 611 intended to encircle the anal orifice. The hollow attachment member 600 has an attachment portion or attachment flange 620 intended to be used to attach the member to the peri-anal skin. A tubular/elongated section with a wall 605 is connected to the attachment portion 620. At the other end of the elongated section 605 there is a connecting element 630 suitable for being connected to a conduit and via the conduit to a collection bag. The connecting element comprises a second opening 631.

Figure 9b discloses a top view of the hollow attachment member 600 of figure 9a. Here it can be seen that the attachment member has an oval shape when seen from the top. This makes the attachment member fit better to the peri-anal skin between the buttocks of the user.

Figures 10a and 10b disclose views of the hollow attachment member 600 from figures 9a and 9b and an adhesive member 700. In this example, the adhesive member 700 is in the form of an adhesive laminate attached to the attachment portion 620 of the hollow attachment member 600.

Figure 11 discloses a cross sectional view of a hollow attachment member 600 from figure 10a and 10b and an adhesive member 700. Furthermore, figure 11 discloses an application structure or applicator 800. The application structure encircles a portion of the tubular section 605 of the attachment member and supports the position of the attachment flange 620 of the hollow attachment member 600. The application structure 800 is intended to be used to apply and gently press the attachment portion 620 of the hollow attachment member 600 (and consequently also the adhesive member (700)) against the peri-anal skin. The application structure comprises a finger grip acting as a finger receiving portion 810.

Figure 12 discloses a cross sectional view of an example of the hollow attachment member 600 from figures 10a, 10b and an adhesive member 700. Furthermore, an application structure 700 is shown. The application structure comprises a chamber 820 in which a portion of the hollow attachment member comprising the connecting element 630 is positioned. The application structure 800 in this embodiment acts as both application structure and as a part of the packaging material. After application of the attachment portion 620 of the hollow attachment member 600 - and consequently the adhesive member 700 against the peri-anal skin, the application structure 800 is removed. The application structure comprises a finger grip acting as a finger receiving portion 810.

Figures 13a and 13b discloses cross sectional views of an example of the hollow attachment member 600 and an adhesive member 700 from figures 10a and 10b. Furthermore, an application structure 800 is shown. The application structure 800 in this example is formed as a portion of the connector 900. The connector 900 consists of an outer shell 901 and an inner element 902. The outer shell 902 acts as the application structure 800. The connector parts 901 and 902 form a space 911 for receiving the connecting element 630 of the hollow attachment member 600. A detachable portion 660 of the hollow attachment member 600 is detachably attached to the top of the application structure 800. The attached part comprises the attachment portion 620 and defines the first opening 611.

Fig. 13a discloses a cross sectional view where the detachable portion 660 of the hollow attachment member (HAM) 600 is attached to the HAM-receiving top 870 of the application structure 800. Fig. 13b discloses a cross sectional view where the detachable portion 660 of the hollow attachment member (HAM) 600 is detached from the HAM-receiving top 870 of the application structure 800. The HAM-receiving top 870 is in this example a part of the outer shell 902 of the connector 900. In other not disclosed examples, the HAM-receiving top 870 is designed to be a part of the inner element 901. The outer shell 902 of the application structure also comprises a finger grip acting as a finger receiving portion 810.

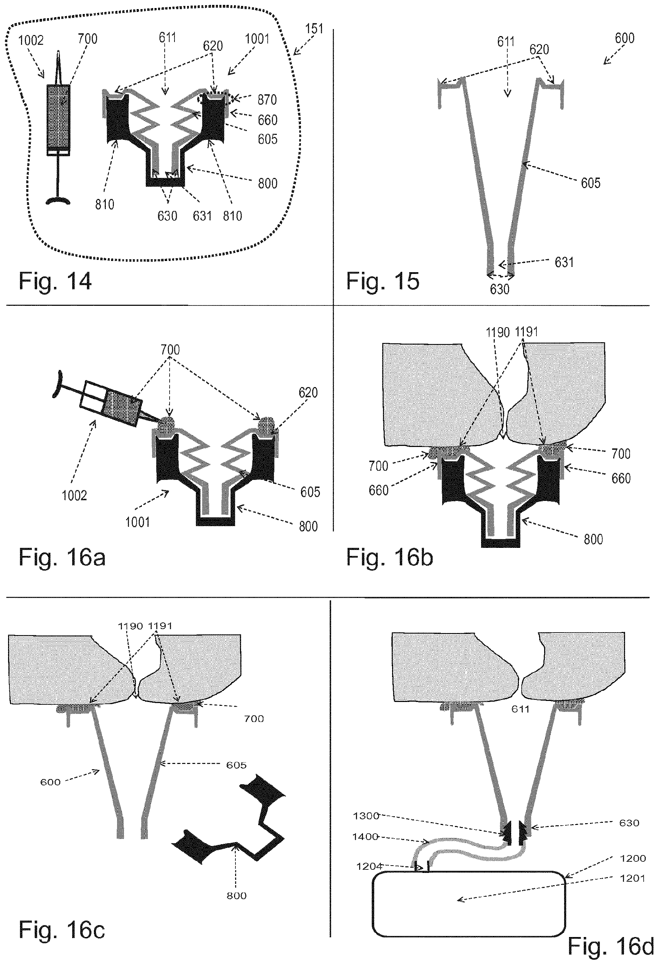

Figure 14 discloses a cross sectional view of an example of a faecal collecting system in the form of a kit of parts according to the present invention comprising the hollow attachment member 600, an adhesive member 700 and an application structure 800. In this example, the adhesive member 700 is in the form of a liquid curable adhesive, supplied in a syringe. The Faecal collecting system comprises two separate parts, a first part 1001 comprising the hollow attachment member 600 and the application structure 800. The second part 1002 is the syringe comprising the adhesive member 700.

The attachment portion 620 is a recess encircling the first opening 611 in the hollow attachment member 600. In this example, the application structure 800 acts as a part of the primary packaging material. In figure 14a, the chamber 820 of the application structure 800 contains the connecting element 630 of the hollow attachment member (HAM) 600, but also a large portion of the rest of the member including the tubular section 605 and the second opening 631. The tubular section is folded into the chamber 820 of the application structure. The application structure comprises a finger grip acting as the finger receiving portion 810. The application structure comprises the HAM-receiving top 870 in which the recess of the hollow attachment member 620 is positioned. As the application structure in this example is intended to be removed from the hollow attachment member, it may be important to reduce the risk that the adhesive member 700 gets into contact with the application structure 800. This is done by elongating an outer flap of the hollow attachment member 600. The outer flap also acts as a detachable portion 660 of the hollow attachment member.

Figure 15 discloses a cross sectional view of the hollow attachment member 600 in figure 14. It may be noted, that the actual length of the attachment member is significantly longer than the application structure 800.

Figure 16a-16d discloses schematic cross sectional views of the example shown in figure 14. Figure 16a-16d is a series of pictures illustrating how the faecal collecting system is attached to the peri-anal skin. Figure 16a illustrates the application of the liquid curable adhesive 700 to the attachment portion 620 of the hollow attachment member 600 while the hollow attachment member 600 is positioned in the application structure 800.

Figure 16b illustrates how the system, when the adhesive 700 has been applied on the attachment member 600, is positioned on the peri-anal skin 1191 surrounding the anal orifice 1190. The liquid adhesive 700 will migrate into the skin folds creating a liquid tight seal and cure. Furthermore, the detachable portion 660 of the hollow attachment member protects the application structure 800 from contacting the adhesive 700.

Figure 16c illustrates how the tubular section 605 of the attachment member 600 unfolds upon removal of the application structure 800. The application structure 800 is removed when the adhesive 700 has cured sufficiently.

Figure 16d illustrates how the hollow attachment member 600 may be connected to a faecal collecting bag 1200 with an inlet 1204 and a space 1201 to receive the stool. The connecting element 630 is attached to a connector 1300 comprising a tube 1400. The tube 1400 is attached to the inlet 1204 of the collecting bag. The user may now change parts of the faecal collecting system without a need to change the entire system.

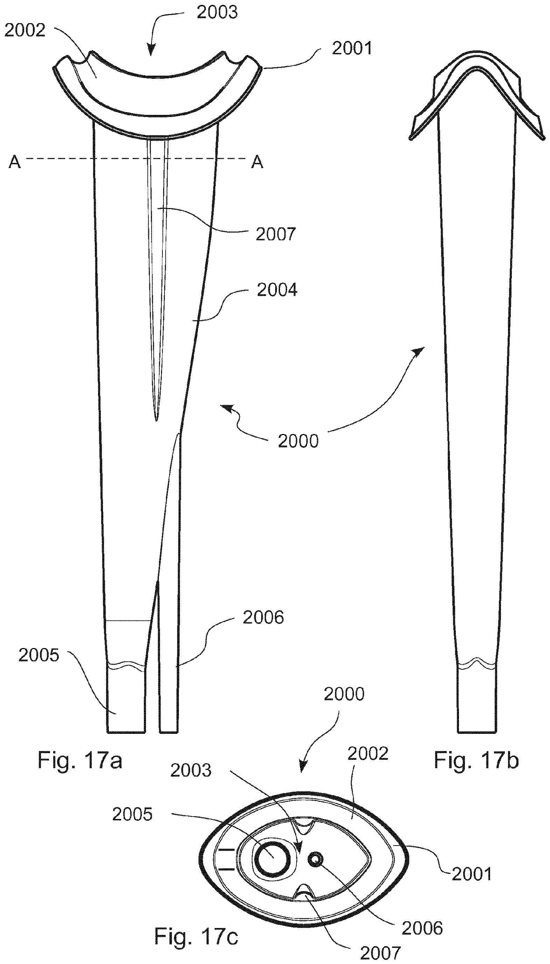

Figures 17a-17c show different views of one embodiment of an attachment member 2000 according to the current invention. The attachment member 2000 is made from a flexible plastic material which is suitable for being in contact with human skin for a longer period of time. One example of a suitable material is a silicone material of shore A50 or less.

The attachment member is approximately 18cm long in the current embodiment and has an average wall thickness of around 0.3cm. The attachment member has an attachment flange 2001 with a groove 2002. Prior to attachment to a user, an adhesive (not shown) can be applied in the groove. The attachment flange and the groove encircle an opening 2003 which leads to a hollow elongated portion 2004.

A hose (not shown) can be connected to a connector 2005 at the end of the elongated portion opposite to the attachment flange. Furthermore, the attachment member of the current invention has an inlet 2006 for connecting to a liquid/water hose for rinsing the attachment member or for introducing liquid into the anus for loosening hard stool inside the rectum.

In the understanding of the current specification, the elongated portion below the line AA would be considered to be the "conduit" together with the hose connected to the connector 2005.

The attachment member of the current embodiment also has ribs 2007 which strengthen the upper portion of the attachment member and prevent it from collapsing during use. This helps to reduce the risk of the attachment member collapsing and blocking the flow of faeces.

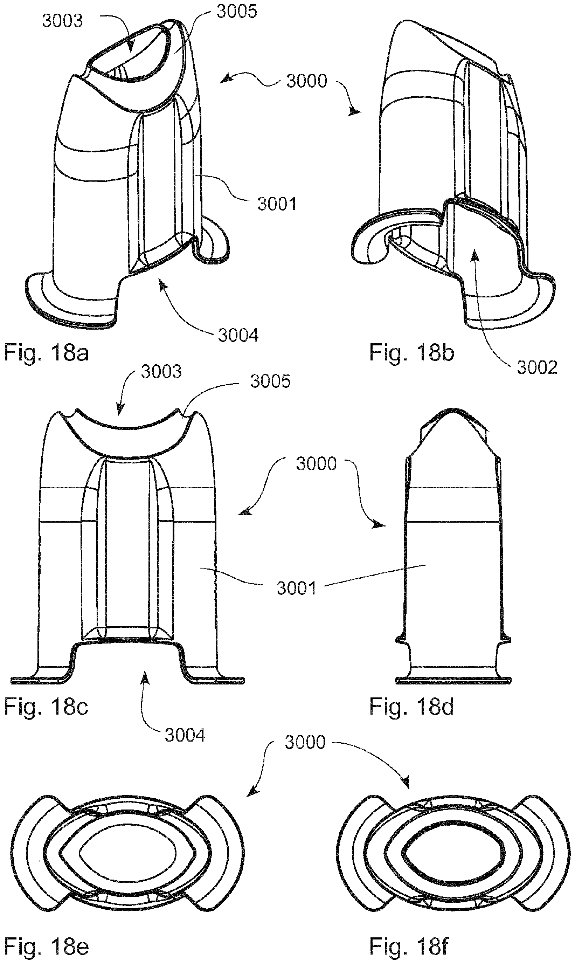

Figures 18a-18f show different views of one embodiment of an applicator 3000 for applying the attachment member 2000 of figures 17a-17c. The applicator 3000 has a handle portion 3001 with a hollow portion 3002. At one end of the handle portion is a first opening 3003 which is in connection with the hollow portion. In this embodiment, there is also an opening 3004 at the end of the handle portion opposite to the first opening 3003.

Encircling the first opening 3003 is a support flange 3005 which is formed complementary to the lower side of the attachment flange 2001 of the attachment member 2000 of figures 17a-17c. In order to apply the attachment member, the elongated portion of the attachment member is inserted into the first opening so that the elongated portion is arranged inside the hollow portion and the lower surface of the attachment flange is supported by the support flange 3005 of the applicator.

Due to the flexible nature of the attachment member, the elongated portion of the attachment member can be folded up inside the hollow portion of the applicator. Furthermore, a hose can be attached to the connector of the attachment member and also folded up inside the applicator. A lid (not shown) can be inserted into the second opening 3004 to secure the conduit inside the applicator. In this way, the applicator can be used as a form of packaging to protect the conduit.

When it is desired to use the system, an adhesive can be applied in the groove of the attachment member, after which the attachment member can be pressed into contact with the peri-anal skin surrounding the anus of the user via the applicator. The person applying the system to the user can just hold onto the applicator without having to contact the user as much as with prior art systems. The applicator can then be drawn back from the attachment member, along the conduit, leaving the attachment member connected to the user.

In one embodiment of the system, the applicator can be pulled back along the conduit until the applicator reaches the end of the conduit, then the applicator can be removed entirely from the system and discarded. The end of the conduit can then be connected to the collection bag.

In another embodiment of the system, the applicator could be provided with a connector which connects to the end of the conduit such that the applicator supports the end of the conduit. In this case, the applicator could also be provided with a hanger or other form of supporting element which could be connected to a hospital bed to support the end of the conduit and/or the collection bag. For example, a hook could be integrated into the applicator such that the applicator could be hung on the frame at the end of a hospital bed.

In another embodiment, the end of the conduit could be connected to a connector element which can be connected to the collection bag. The connector element could be provided with means which make it possible to connect the connector element to the second opening of the applicator. This would secure the end of the conduit with respect to the applicator during storage. Furthermore, in one embodiment, the connector element could be provided with a support element as described above to support the collection bag with respect to a hospital bed or similar during use.

In one embodiment of the system, the applicator is large enough and/or the collection bag is small/flexible enough so that the collection bag can be stored inside the applicator prior to use.

In addition to making application of the attachment member to the user easier, the applicator can also be used when the system is attached to the user to make emptying the conduit easier. In many cases, faeces will collect in the conduit and not reach the collection bag. In this case, a caretaker will need to help empty the conduit. Typically this is done by "stroking" along the length of the conduit to push the faeces along the conduit and towards the collection bag. During such an emptying procedure, there is a risk that the attachment member is detached from the user resulting in leakage. However, with the applicator, this can be prevented. The applicator can be pushed up along the conduit until it again is in contact with the bottom surface of the attachment flange of the attachment member. The applicator can then be pressed towards the user so that the attachment flange is held pressed up against the peri anal skin of the user. The caretaker can then easily stroke the faeces in the conduit into the collection bag with a much reduced risk of leakage.

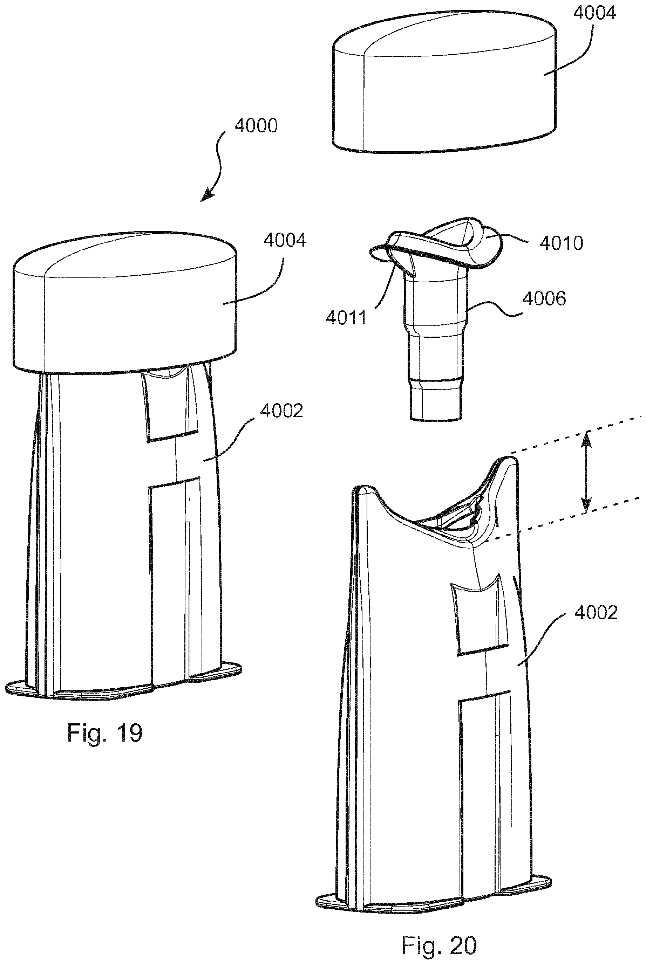

Figures 19 to 25 show different views of another embodiment 4000 of an applicator and attachment member. In this case, the figures mostly show an applicator 4002, a lid 4004 and an attachment member 4006. Many details and usage are similar to previously described embodiments and only a brief description of this embodiment is therefore provided here.

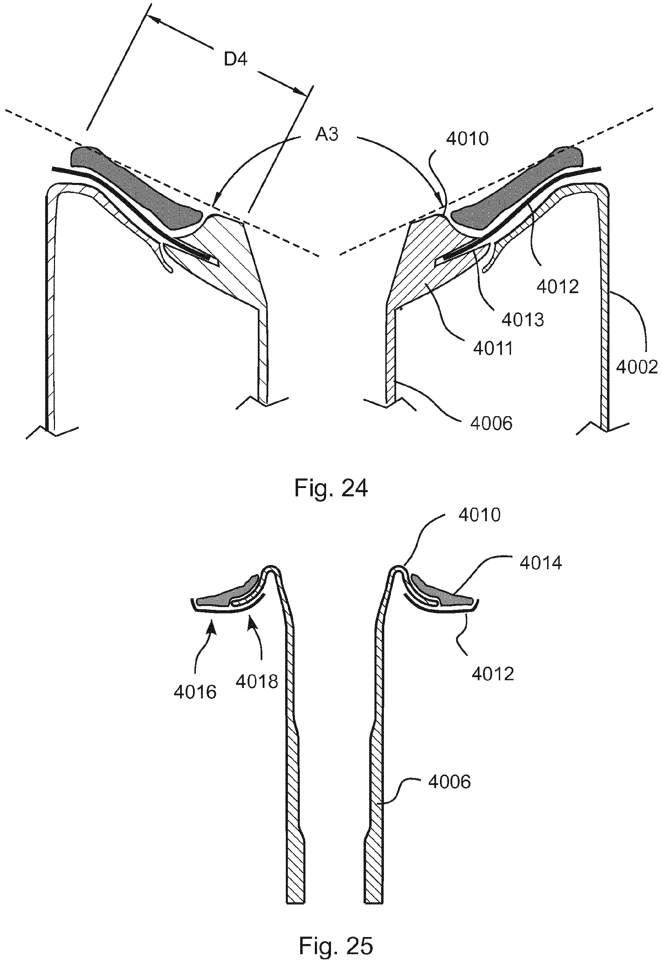

It should be noted that in figures 20, 21, 22, 23 only a portion of the attachment member 4006 is shown. In figures 24 and 25 the complete attachment member is shown. In this respect, it is noted that the attachment member in this embodiment, see figures 24 and 25, comprises a moulded silicon portion 4010, a non-woven flexible sheet portion 4012 and an adhesive 4014. The outer portion 4016 of the attachment flange is therefore very flexible due to the non-woven sheet while the inner portion 4018 of the flange is stiffer and supports the opening of the attachment flange from collapsing. In this embodiment, the two portions of the flange are provided as two separate elements joined together by an adhesive. However, in another embodiment (not shown), the inner and outer portions could be manufactured via a single injection moulding operation where the outer portion is thinner than the inner portion. This will have a similar effect. In another embodiment (not shown) instead of a non-woven outer portion, a foil could be used which is inserted into the injection mould prior to moulding the remaining portion of the attachment flange, similar to In Mould Label (or IML) procedures.

In addition to the two part assembly of the attachment flange, the attachment member of the current embodiment comprises a vertically arranged supporting flange 4011 arranged at both the dorsal and ventral portions of the attachment flange. The supporting flanges 4011 help to push the dorsal and ventral portions into effective contact between the attachment flange and the Perineum and the Crena Ani during application. A slit 4013 is made in the supporting flanges to allow the non woven sheet 4012 to properly engage the moulded portion of the attachment flange. A slit also ensures that bending forces on the conduit are not transmitted to the attachment flange via the supporting flanges. The flanges with the slit only act to push the flange into contact with the perianal skin, not pull it way.

Another difference between the attachment member of this embodiment and the previous embodiment, is that the attachment flange is wider, see D4 in figure 24. In this embodiment, the attachment flange has a width of around 22mm all the way around the opening of the attachment member. Another difference is that the skin contacting surfaces of the attachment flange at the dorsal and ventral portions of the attachment flange form an angle to each other of around 130 degrees in this embodiment, see A3 in figure 24. This angle allows the dorsal and ventral portions to better contact the Crena Ani and Perineum portions of the user more effectively.

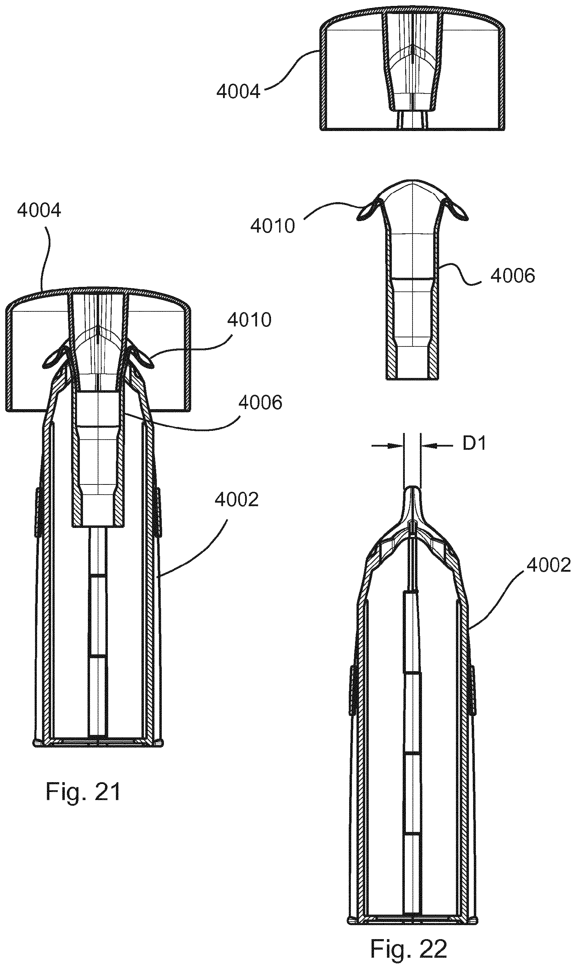

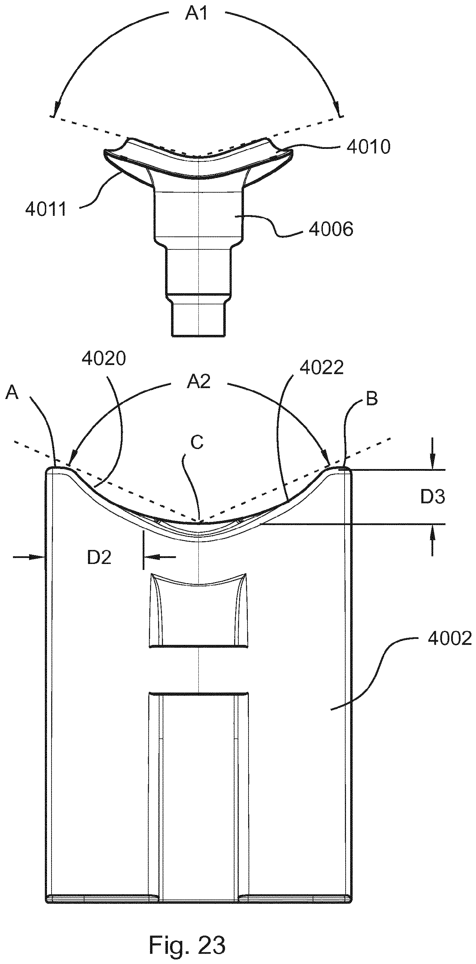

One main difference between the applicator 4002 shown in figures 19-25 and the applicator 3000 shown in figures 18a-18f is that the dorsal portion 4020 and ventral portion 4022 of the applicator have been made longer (see D2 in figure 23) and thinner (see D1 in figure 22). In this way, when the applicator is applied to the user, the pressure applying surface of the applicator can apply pressure to the Perineum and the Crena Ani.

Furthermore, the pressure applying surface of the applicator has been elevated dorsally and ventrally with respect to the central portion (see D3 in figure 23). As with the attachment member, the elevation difference between the dorsal and ventral portions and the central portion creates an angle between the dorsal and ventral portions (see A2 in figure 23). In this embodiment, the angle between the lines AC and BC in figure 23 is approximately 135 degrees.

It should be noted that in this embodiment, the applicator is completely symmetric with regards to the dorsal/ventral portions. However in another embodiment (not shown), it could be that the dorsal portion is higher than the ventral portion.

In the above embodiments, applicators have been shown which are plastic injection moulded parts which have a pressure applying surface which extends around the entire opening. Thereby the applicator can be used to apply pressure to the attachment flange all the way around the opening of the attachment member. However, it could also be imagined that a more simple applicator was provided in some embodiments. For example, a bent metal rod of 4mm in diameter could be bent into a suitable shape, having a proximal handle portion and two distal pressure applying portions which engage with the dorsal and ventral portions of the attachment flange, but which do not contact the side portions of the attachment flange. The conduit could be arranged between the two distal pressure applying portions. With this applicator, the attachment flange could be pressed into contact with the Perineum and the Crena Ani. Once the attachment flange was in contact with the Perineum and the Crena Ani, the sides of the attachment flange could be manually folded out to engage the skin of the user.

It is to be noted that the figures and the above description have shown the example embodiments in a simple and schematic manner. Many of the specific mechanical details have not been shown since the person skilled in the art should be familiar with these details and they would just unnecessarily complicate this description. For example, the specific materials used and the specific fabrication techniques have not been described in detail since it is maintained that the person skilled in the art would be able to find suitable materials and suitable processes to manufacture the products according to the current invention.

## Claims

1. Faecal collecting system (1) arranged to collect faecal waste from a user, said system comprising:
a. an attachment member (10) arranged to be attached to the user, said attachment member comprising an opening (18) which is arranged to be in fluid communication with the anus (4) of the user and an attachment flange (16) which is arranged to encircle said opening and which is arranged to be attached to the peri-anal skin (2) surrounding the anus of the user such that a fluid tight seal is established between the attachment flange and the peri-anal skin of the user entirely around the anus of the user,
b. a collection bag (14) for collecting the faecal output, and
c. a hollow conduit (12) having a first end (22) and a second end (24), said first end connected to said attachment member and being in fluid communication with the opening of said attachment member and said second end being in fluid communication with said collection bag,
d. said attachment flange (16) has an outer width which is larger than the width of the conduit near the first end (22) of the conduit, **characterized in that**
e. said conduit is at least 30 cm in length,
f. said conduit has an average diameter of less than 8 cm,
g. said attachment flange is provided with a skin friendly adhesive (700) on a skin contacting surface of said attachment flange and **in that**
h. the flange portion and the adhesive are sufficiently flexible and elastic to be able to adapt to varying anatomies.

2. A faecal collecting system (1) according to claim 1, **characterized in that** said conduit (12) is provided with anti-kinking and/or anti-collapsing means.

3. A faecal collecting system (1) according to any one of claims 1 to 2,
**characterized in that** the attachment flange (16) comprises a first portion (4018) closest to the opening (18) of the attachment member (10) and a second portion (4016) further away from the opening than the first portion, said first portion being stiffer than said second portion.

4. A faecal collecting system (1) according to any one of claims 1 to 3, **characterized in that** the attachment flange (2001) is formed as a 3D formed structure.

5. A faecal collecting system (1) according to claim 4, **characterized in that** a dorsal portion and/or a ventral portion of the attachment flange (16) is/are higher than a central portion of the attachment flange.

6. A faecal collecting system (1) according to any one of claims 1 to 5, **characterized in that** a dorsal portion and/or a ventral portion of the attachment flange (16) extend dorsally (D4) at least 12 mm, at least 15mm, at least 18mm or at least 20mm from the dorsal/ventral portion of the opening respectively.

7. A faecal collecting system (1) according to any one of claims 1 to 5, **characterized in that** said system comprises a conduit connection member (1204) which allows the conduit (12) to be detachably connected to the collection bag (14).

8. A faecal collecting system (1) according to any one of claims 1 to 7, **characterized in that** said attachment flange (2001) is provided with a groove (2002) on the skin contacting surface of said attachment flange, said groove encircling the opening (2003), and said groove being filled with a liquid adhesive (700).

9. A faecal collecting system (1) according to any one of claims 1 to 8, **characterized in that** the thickness of the adhesive layer (4014) is greater than 1 mm, greater than 2mm or greater than 3mm.

10. A faecal collecting system (1) according to any one of claims 1-9, **characterized in that** said system further comprises an applicator (3000), said applicator comprising a handle portion (3001), an opening (3003) arranged at one end of the handle portion, said opening being arranged to allow the conduit (12) to pass through the opening and a pressure applying surface (3005) arranged at the opening, said pressure applying surface being arranged to be suitable for pressing the attachment flange (2001) of the attachment member (2000) against the perianal skin of the Perineum and the Crena Ani of the user.

11. A faecal collecting system (1) according to claim 10, **characterized in that** a dorsal portion (4020) and/or a ventral portion (4022) of the pressure applying surface (4020,4022) of the applicator (4002) has a top point (A,B) elevated at least 5 mm, at least 7mm, at least 9mm or at least 11mm in the cranial direction from the portion (C) of the applicator adapted to receive the opening of said attachment member.

12. A faecal collecting system (1) according to claim 10 or 11, **characterized in that** a ventral (4020) and a dorsal portion (4022) of the pressure applying surface (4020,4022) is less than 13mm wide, less than 11mm wide, less than 8mm wide or less than 6mm wide.

13. A faecal collecting system (1) according to any one of claims 10 to 12, **characterized in that** an angle (A2) between a path along a dorsal portion (4020) of the pressure applying surface (4020,4022) along the central plane of the applicator and a path along a ventral portion (4022) of the pressure applying surface along the central plane of the applicator (4002) is less than 170 degrees, less than 155 degrees, or less than 140 degrees.

14. A faecal collecting system (1) according to any one of claims 10-13, **characterized in that** the applicator (4002) comprises a ventral top point (B) on a ventral portion (4022) of the pressure applying surface; a dorsal top point (A) on a dorsal portion (4020) of the pressure applying surface; a middle top point (C) on a middle portion of the pressure applying surface in the ventral-dorsal direction, said middle top point being lower than the dorsal top point and/or the ventral top point; and where the angle (A2) formed between the lines CA and CB is between 115 and 170 degrees, between 120 and 160 degrees, between 125 and 150 degrees or any combination of the endpoints of these intervals.

15. A faecal collecting system (1) according to any one of claims 1 to 14, **characterized in that** said system comprises a fastening structure (201), said fastening structure comprising a waistband (210) and a supporting strap arrangement (221,222) arranged to press the attachment flange (310) of the attachment member (300) against the peri-anal skin (2) of the user.

## Patentansprüche

1. Fäkaliensammelsystem (1), das zum Sammeln von Fäkalien eines Benutzers angeordnet ist, wobei das System Folgendes umfasst:
a. ein Befestigungselement (10), das so angeordnet ist, dass es am Benutzer befestigt werden kann, wobei das Befestigungselement eine Öffnung (18) umfasst, die so angeordnet ist, dass sie in strömungstechnischer Kommunikation mit dem Anus (4) des Benutzers ist, und einen Befestigungsflansch (16), der so angeordnet ist, dass er die Öffnung umgibt und so angeordnet ist, dass er an der den Anus des Benutzers umgebenden perianalen Haut (2) befestigt werden kann, sodass eine flüssigkeitsdichte Abdichtung zwischen dem Befestigungsflansch und der perianalen Haut des Benutzers um den Anus des Benutzers vollständig hergestellt wird,
b. einen Sammelbeutel (14) zum Sammeln des Fäkalienausstoßes, und
c. eine Hohlleitung (12), die ein erstes Ende (22) und ein zweites Ende (24) aufweist, wobei das erste Ende mit dem Befestigungselement verbunden ist und in strömungstechnischer Kommunikation mit der Öffnung des Befestigungselements ist, und das zweite Ende in strömungstechnischer Kommunikation mit dem Sammelbeutel ist,
d. wobei der Befestigungsflansch (16) eine äußere Breite aufweist, die größer als die Breite der Leitung in der Nähe des ersten Endes (22) der Leitung ist, **dadurch gekennzeichnet, dass**
e. die Leitung mindestens 30 cm lang ist,
f. die Leitung einen durchschnittlichen Durchmesser von weniger als 8 cm aufweist,
g. der Befestigungsflansch mit einem hautfreundlichen Klebstoff (700) auf einer die Haut berührenden Oberfläche des Befestigungsflansches bereitgestellt ist, und dadurch, dass
h. der Flanschabschnitt und der Klebstoff ausreichend flexibel und elastisch sind, um sich an unterschiedliche Anatomien anpassen zu können.

2. Fäkaliensammelsystem (1) nach Anspruch 1, **dadurch gekennzeichnet, dass** die Leitung (12) mit einem Knickschutz- und/oder Einfallschutzmittel bereitgestellt ist.

3. Fäkaliensammelsystem (1) nach einem der Ansprüche 1 bis 2, **dadurch gekennzeichnet, dass** der Befestigungsflansch (16) einen ersten Abschnitt (4018), der der Öffnung (18) des Befestigungselements (10) am nächsten liegt, und einen zweiten Abschnitt (4016), der weiter von der Öffnung entfernt ist als der erste Abschnitt, umfasst, wobei der erste Abschnitt steifer als der zweite Abschnitt ist.

4. Fäkaliensammelsystem (1) nach einem der Ansprüche 1 bis 3, **dadurch gekennzeichnet, dass** der Befestigungsflansch (2001) als 3D-geformte Struktur ausgebildet ist.

5. Fäkaliensammelsystem (1) nach Anspruch 4, **dadurch gekennzeichnet, dass** ein dorsaler Abschnitt und/oder ein ventraler Abschnitt des Befestigungsflansches (16) höher als ein zentraler Abschnitt des Befestigungsflansches ist/sind.

6. Fäkaliensammelsystem (1) nach einem der Ansprüche 1 bis 5, **dadurch gekennzeichnet, dass** ein dorsaler Abschnitt und/oder ein ventraler Abschnitt des Befestigungsflansches (16) sich jeweils mindestens 12 mm, mindestens 15 mm, mindestens 18 mm oder mindestens 20 mm dorsal (D4) von dem dorsalen/ventralen Abschnitt der Öffnung erstrecken.

7. Fäkaliensammelsystem (1) nach einem der Ansprüche 1 bis 5, **dadurch gekennzeichnet, dass** das System ein Leitungsverbindungselement (1204) umfasst, das der Leitung (12) ermöglicht, lösbar mit dem Sammelbeutel (14) verbunden zu werden.

8. Fäkaliensammelsystem (1) nach einem der Ansprüche 1 bis 7, **dadurch gekennzeichnet, dass** der Befestigungsflansch (2001) mit einer Nut (2002) auf der die Haut berührenden Oberfläche des Befestigungsflansches bereitgestellt ist, wobei die Nut die Öffnung (2003) umgibt und die Nut mit einem flüssigen Klebstoff (700) gefüllt ist.

9. Fäkaliensammelsystem (1) nach einem der Ansprüche 1 bis 8, **dadurch gekennzeichnet, dass** die Dicke der Klebstoffschicht (4014) größer als 1 mm, größer als 2 mm oder größer als 3 mm ist.

10. Fäkaliensammelsystem (1) nach einem der Ansprüche 1-9, **dadurch gekennzeichnet, dass** das System weiter einen Applikator (3000) umfasst, wobei der Applikator einen Griffabschnitt (3001), eine an einem Ende des Griffabschnitts angeordnete Öffnung (3003), wobei die Öffnung so angeordnet ist, dass sie der Leitung (12) ermöglicht, durch die Öffnung hindurchzugehen, und eine bei der Öffnung angeordnete Druckaufbringungsfläche (3005), wobei die Druckaufbringungsfläche so angeordnet ist, dass sie zum Drücken des Befestigungsflansches (2001) des Befestigungselements (2000) gegen die perianale Haut des Perineums und der Gesäßspalte des Benutzers geeignet ist, umfasst.

11. Fäkaliensammelsystem (1) nach Anspruch 10, **dadurch gekennzeichnet, dass** ein dorsaler Abschnitt (4020) und/oder ein ventraler Abschnitt (4022) der Druckaufbringungsfläche (4020, 4022) des Applikators (4002) einen oberen Punkt (A, B) aufweist, der um mindestens 5 mm, mindestens 7 mm, mindestens 9 mm oder mindestens 11 mm in der kranialen Richtung vom Abschnitt (C) des Applikators erhöht ist, der dazu geeignet ist, die Öffnung des Befestigungselements aufzunehmen.

12. Fäkaliensammelsystem (1) nach Anspruch 10 oder 11, **dadurch gekennzeichnet, dass** ein ventraler (4020) und ein dorsaler Abschnitt (4022) der Druckaufbringungsfläche (4020, 4022) weniger als 13 mm breit, weniger als 11 mm breit, weniger als 8 mm breit oder weniger als 6 mm breit sind.

13. Fäkaliensammelsystem (1) nach einem der Ansprüche 10 bis 12, **dadurch gekennzeichnet, dass** ein Winkel (A2) zwischen einem Pfad entlang eines dorsalen Abschnitts (4020) der Druckaufbringungsfläche (4020, 4022) entlang der zentralen Ebene des Applikators und ein Pfad entlang eines ventralen Abschnitts (4022) der Druckaufbringungsfläche entlang der zentralen Ebene des Applikators (4002) weniger als 170 Grad, weniger als 155 Grad oder weniger als 140 Grad beträgt.

14. Fäkaliensammelsystem (1) nach einem der Ansprüche 10-13, **dadurch gekennzeichnet, dass** der Applikator (4002) einen ventralen oberen Punkt (B) auf einem ventralen Abschnitt (4022) der Druckaufbringungsfläche umfasst; einen dorsalen oberen Punkt (A) auf einem dorsalen Abschnitt (4020) der Druckaufbringungsfläche; einen mittleren oberen Punkt (C) auf einem mittleren Abschnitt der Druckaufbringungsfläche in der ventral-dorsalen Richtung, wobei der mittlere obere Punkt tiefer als der dorsale obere Punkt und/oder der ventrale obere Punkt ist; und wobei der zwischen den Linien CA und CB gebildete Winkel (A2) zwischen 115 und 170 Grad, zwischen 120 und 160 Grad, zwischen 125 und 150 Grad oder einer beliebigen Kombination der Endpunkte dieser Intervalle beträgt.

15. Fäkaliensammelsystem (1) nach einem der Ansprüche 1 bis 14, **dadurch gekennzeichnet, dass** das System eine Befestigungsstruktur (201) umfasst, wobei die Befestigungsstruktur einen Bund (210) und eine Stützgurtanordnung (221, 222) umfasst, die angeordnet sind, um den Befestigungsflansch (310) des Befestigungselements (300) gegen die perianale Haut (2) des Benutzers zu drücken.

## Revendications

1. Système de collecte de matières fécales (1) agencé pour collecter des déchets fécaux d'un utilisateur, ledit système comprenant :
a. un élément de fixation (10) agencé pour être fixé à l'utilisateur, ledit élément de fixation comprenant une ouverture (18) qui est agencée pour être en communication fluidique avec l'anus (4) de l'utilisateur et une bride de fixation (16) qui est agencée pour encercler ladite ouverture et qui est agencée pour être fixée à la peau péri-anale (2) entourant l'anus de l'utilisateur de telle sorte qu'un joint étanche aux fluides soit établi entre la bride de fixation et la peau péri-anale de l'utilisateur entièrement autour de l'anus de l'utilisateur,
b. un sac de collecte (14) pour collecter les excréments fécaux et
c. un conduit creux (12) présentant une première extrémité (22) et une seconde extrémité (24), ladite première extrémité étant reliée au dit élément de fixation et étant en communication fluidique avec l'ouverture dudit élément de fixation et ladite seconde extrémité étant en communication fluidique avec ledit sac de collecte,
d. ladite bride de fixation (16) présente une largeur externe qui est plus importante que la largeur du conduit près de la première extrémité (22) du conduit, **caractérisé en ce que**
e. ledit conduit présente une longueur d'au moins 30 cm,
f. ledit conduit présente un diamètre moyen inférieur à 8 cm,
g. ladite bride de fixation est pourvue d'un adhésif respectueux de la peau (700) sur une surface venant en contact avec la peau de ladite bride de fixation et **en ce que**
h. la partie bride et l'adhésif sont suffisamment flexibles et élastiques pour pouvoir s'adapter à différentes anatomies.

2. Système de collecte de matières fécales (1) selon la revendication 1, **caractérisé en ce que** ledit conduit (12) est pourvu de moyens anti-vrillage et/ou anti-chute.

3. Système de collecte de matières fécales (1) selon l'une quelconque des revendications 1 à 2, **caractérisé en ce que** la bride de fixation (16) comprend une première partie (4018) la plus proche de l'ouverture (18) de l'élément de fixation (10) et une seconde partie (4016) plus éloignée de l'ouverture que la première partie, ladite première partie étant plus rigide que ladite seconde partie.

4. Système de collecte de matières fécales (1) selon l'une quelconque des revendications 1 à 3, **caractérisé en ce que** la bride de fixation (2001) est formée sous la forme d'une structure formée en 3D.

5. Système de collecte de matières fécales (1) selon la revendication 4, **caractérisé en ce qu'**une partie dorsale et/ou une partie ventrale de la bride de fixation (16) est/sont plus hautes qu'une partie centrale de la bride de fixation.

6. Système de collecte de matières fécales (1) selon l'une quelconque des revendications 1 à 5, **caractérisé en ce qu'**une partie dorsale et/ou une partie ventrale de la bride de fixation (16) s'étend de manière dorsale (D4) sur au moins 12 mm, au moins 15 mm, au moins 18 mm ou au moins 20 mm à partir de la partie dorsale/ventrale de l'ouverture respectivement.

7. Système de collecte de matières fécales (1) selon l'une quelconque des revendications 1 à 5, **caractérisé en ce que** ledit système comprend un élément de raccordement de conduit (1204) qui permet au conduit (12) d'être raccordé de manière amovible au sac de collecte (14).

8. Système de collecte de matières fécales (1) selon l'une quelconque des revendications 1 à 7, **caractérisé en ce que** ladite bride de fixation (2001) est pourvue d'une rainure (2002) sur la surface en contact avec la peau de ladite bride de fixation, ladite rainure encerclant l'ouverture (2003) et ladite rainure étant remplie d'un adhésif liquide (700).

9. Système de collecte de matières fécales (1) selon l'une quelconque des revendications 1 à 8, **caractérisé en ce que** l'épaisseur de la couche adhésive (4014) est plus grande que 1 mm, plus grande que 2 mm ou plus grande que 3 mm.

10. Système de collecte de matières fécales (1) selon l'une quelconque des revendications 1-9, **caractérisé en ce que** ledit système comprend en outre un applicateur (3000), ledit applicateur comprenant une partie poignée (3001), une ouverture (3003) agencée au niveau d'une extrémité de la partie poignée, ladite ouverture étant agencée pour permettre au conduit (12) de passer par l'ouverture et une surface d'application de pression (3005) agencée au niveau de l'ouverture, ladite surface d'application de pression étant agencée pour être appropriée pour appuyer sur la bride de fixation ( 2001) de l'élément de fixation (2000) contre la peau périanale du perineum et du Crena Ani de l'utilisateur.

11. Système de collecte de matières fécales (1) selon la revendication 10, **caractérisé en ce qu'**une partie dorsale (4020) et/ou une partie ventrale (4022) de la surface d'application de pression (4020, 4022) de l'applicateur (4002) présente un point supérieur (A, B) surélevé d'au moins 5 mm, d'au moins 7 mm, d'au moins 9 mm ou d'au moins 11 mm dans la direction crânienne à partir de la partie (C) de l'applicateur adaptée pour recevoir l'ouverture dudit élément de fixation.

12. Système de collecte de matières fécales (1) selon la revendication 10 ou 11, **caractérisé en ce qu'**une partie ventrale (4020) et une partie dorsale (4022) de la surface d'application de pression (4020, 4022) présentent une largeur inférieure à 13 mm, une largeur inférieure à 11 mm, une largeur inférieure à 8 mm ou une largeur inférieure à 6 mm.

13. Système de collecte de matières fécales (1) selon l'une quelconque des revendications 10 à 12, **caractérisé en ce qu'**un angle (A2) entre un trajet le long d'une partie dorsale (4020) de la surface d'application de pression (4020, 4022) le long du plan central de l'applicateur et un trajet le long d'une partie ventrale (4022) de la surface d'application de pression le long du plan central de l'applicateur (4002) est inférieur à 170 degrés, inférieur à 155 degrés ou inférieur à 140 degrés.

14. Système de collecte de matières fécales (1) selon l'une quelconque des revendications 10-13, **caractérisé en ce que** l'applicateur (4002) comprend un point supérieur ventral (B) sur une partie ventrale (4022) de la surface d'application de pression ; un point supérieur dorsal (A) sur une partie dorsale (4020) de la surface d'application de pression ; un point supérieur médian (C) sur une partie médiane de la surface d'application de pression dans la direction ventrale-dorsale, ledit point supérieur médian étant plus bas que le point supérieur dorsal et/ou le point supérieur ventral ; et dans lequel l'angle (A2) formé entre les lignes CA et CB est entre 115 et 170 degrés, entre 120 et 160 degrés, entre 125 et 150 degrés ou toute combinaison des points d'extrémité de ces intervalles.

15. Système de collecte de matières fécales (1) selon l'une quelconque des revendications 1 à 14, **caractérisé en ce que** ledit système comprend une structure de fixation (201), ladite structure de fixation comprenant une ceinture (210) et un agencement de sangles de support (221, 222) agencé pour presser la bride de fixation (310) de l'élément de fixation (300) contre la peau péri-anale (2) de l'utilisateur.
